Europäisches Patentamt

European Patent Office

Office européen des brevets

(11) Publication number: **0 377 916**
**A1**

## EUROPEAN PATENT APPLICATION

(21) Application number: 89203154.3

(51) Int. Cl.⁵: **C07C 305/10, C07C 303/24**

(22) Date of filing: **12.12.89**

| | |
|---|---|
| The applicant has been informed that the priority right claimed is not recognised, since the European application was filed more than 12 months after the previous application (Art. 87 (1) EPC). | (71) Applicant: **STAMICARBON B.V.**<br>**Mijnweg 1**<br>**NL-6167 AC Geleen(NL)** |
| (30) Priority: **09.12.88 NL 8803025** | (72) Inventor: **Smid, Jacob Kornelis**<br>**J.G. Heuthorststraat 61**<br>**NL-7009 CL Doetinchem(NL)** |
| (43) Date of publication of application:<br>**18.07.90 Bulletin 90/29** | Inventor: **Van der Veen, Reinout Herbert**<br>**Goudaweg 17**<br>**NL-6843 EN Arnhem(NL)**<br>Inventor: **Verschuur, Jacob Gerardus** |
| (84) Designated Contracting States:<br>**AT BE CH DE FR GB IT LI NL** | **Ds. van Dorpstraat 21**<br>**NL-7081 BD Gendringen(NL)** |

(54) **Ethersulphates with low dioxane content.**

(57) Process for preparing ethersulphates by virtually complete sulphation of ethoxylated alcohols, using chlorosulphonic acid, and subsequent neutralization, the sulphation being carried out at least from a degree of conversion of 60% in the presence of one or more dioxane-suppressing compound(s) from the group of (thio)-urea and chlorides, sulphates, sulphites, phosphates, (bi)carbonates, glycolates or carbamates of (earth) alkali metals, ammonium or alkyl- and/or alkanolamines in an amount of at least 0.5% (wt) calculated on the amount of ethoxylated alcohol initially present.

EP 0 377 916 A1

# ETHERSULPHATES WITH LOW DIOXANE CONTENT

The invention relates to a process for preparing ethersulphates by virtually complete sulphation of ethoxylated alcohols, using chlorosulphonic acid, and subsequent neutralization. Ethersulphates as mentioned in this application can be referred to by means of the general formula of $[R-(OCH_2CH_2)_n-OSO_3]m_M$, where R is an alcohol residue, $\nu$ a number between 0.5 and 20 indicating the average degree of ethoxylation of the product, m = 1 or 2 and M is a cation. R may also stand for a mixture of alcohol residues, respectively be derived from a fatty acid alkanolamide.

Ethersulphates form an important class of compounds with detergent properties that can be used cheaply and excellently for a very wide variety of applications. Alkylethersulphates, often derived from fatty alcohols, are used in particular, and that especially in the form of sodium, magnesium, ammonium or alkanol-ammonium salt, in shampoos and bubble baths, and in bath and shower soaps. These compounds are used also in domestic detergents such as, for instance, washing-up liquids.

For the sulphation in general and more in particular also for the sulphation of ethoxylated alcohols, in principle two processes are applied on a commercial scale, viz. either sulphation via the $SO_3$ process, or sulphation via the $ClSO_3H$ (chlorosulphonic acid) route. An excellent survey of the chemistry of sulphonation and sulphation is to be found in the still authoritative book by E.E. Gilbert, 'Sulfonation and related reactions', Interscience Publishers, New York, 1965.

It has been found that in the sulphation of etheralcohols according to the customary embodiments of these sulphation reactions an amount of dioxane (1,4-dioxane) is formed as byproduct, which amount is in the order of magnitude of 500 to 3000 ppm calculated on 100% ethersulphate. This 100% amount of ethersulphate will hereinafter be referred to as 100% DS (detergent surfactant).

As in such amounts, calculated on DS, dioxane has until recently been regarded as harmless in shampoos and the like, this content has not been regarded by industry as a disadvantage. Particularly in consequence of the negative publicity in various media regarding dioxane in shampoos (with a strong impact on consumer behaviour), a strong need has developed to reduce the dioxane content in ethersulphates. A hard limit, however, has not yet been given in this respect, as set forth by J. Burford during the 2nd World Surfactants Congress of CESIO in Paris, May 1988.

Most producers of ethersulphates have since achieved a substantial reduction of the dioxane content by a combination of measures clear to the person skilled in the art on the basis of the chemistry of the processes. Such measures are the lowering of the reaction temperature, minimizing the time of reaction and the time of the subsequent neutralization step, using a small stoichiometric short measure of sulphating agent calculated on the ethoxylated alcohol, lowering the water content of the starting products and lowering the content of free polyethyleneglycol in the ethoxylated alcohol.

Despite the progress achieved with these in so far as the lowering of the dioxane content is concerned - a level of about 100 ppm dioxane on 100% DS can be achieved with them - the search for possibilities of further reducing the dioxane content is still going on. It will be clear also that each of the various above-mentioned measures may influence the economy of the process and/or the quality of the resulting product. Thus a lowering of the temperature goes hand in hand with an extension of the time of reaction, which ought to be as short as possible, as indicated. Sulphation with a short measure of sulphating agent is possible to only a limited degree, because, in connection with the desired product quality, a virtually complete conversion of the (nonionic) etheralcohol into the ethersulphate is necessary. A degree of conversion lower than 90% gives an ethersulphate that is unsuited, on account of too high a viscosity and tur bidity, for most applications in detergents, cosmetic products and the like.

It should otherwise be noted that research has shown that the formation of dioxane is stronger as the sulphating reaction proceeds. Up to a conversion of 60-75% the dioxane content remains low, but from a conversion of 75-80% an exponential increase occurs.

In the $SO_3$ process it is indeed possible, at the expense of substantial investments, to achieve a further reduction of the dioxane content. The firm of Ballestra S.p.A. for this purpose developed a so-called 'multitube film reactor', in which, in combination also with the measures referred to hereinbefore, a dioxane content of far below 100 ppm on 100% DS can be reached (see, for instance, the report on the 2nd World Surfactants Congress of CESIO in Paris, May 1988: pp. 236-261), part I.

Such a process requires investments and does not seem usable in a chlorosulphonic acid process. Despite their application of a combination of the dioxane-reducing measures mentioned herein-before, producers of ethersulphates using the chlorosulphonic acid route are thus still in need of a process for preparing ethersulphates having a low dioxane content with virtually complete sulphation of the etheralcohol. Virtually complete sulphation is understood to mean in this connection a conversion into ethersulphate of at least 90%.

The object of the invention therefore is to provide a process for preparing ethersulphates by sulphation of ethoxylated alcohols, using chlorosulphonic acid, and subsequent neutralization, in which process ethersulphate with a low dioxane content is obtained under virtually complete sulphation.

It has now surprisingly been found, and such is the characteristic of the invention, that ethersulphates with a low dioxane content can be prepared by virtually complete sulphation of ethoxylated alcohols, using chlorosulphonic acid, and subsequent neutralization if at least from a degree of conversion of 60% the sulphation is carried out in the presence of one or more dioxane-suppressing compound(s) from the group of (thio)urea and chlorides, sulphates, sulphites, phosphates, (bi)carbonates, glycolates or carbamates of (earth) alkali metals, ammonium or alkyl- and/or alkanolamines in an amount of at least 0.5% (wt) calculated on the amount of ethoxylated alcohol initially present.

The ethersulphates thus obtained have a dioxane content significantly lower than if the sulphation is carried out without one or more of the said compounds being present in the final sulphation stage (from 60% conversion).

For practical reasons preference should be given to the relevant compound(s) being present already from the beginning of the sulphation process, but as stated earlier, the formation of dioxane is strongest in the final stage of the sulphation process and the presence of one or more compound(s) according to the invention is required particularly in that stage to suppress the formation of dioxane. Preference is given to adding the dioxane-suppressing compound(s) to the etheralcohol before metering the chlorosulphonic acid, i.e. before commencement of the sulphation.

Example of compounds having a dioxane-reducing effect according to the invention are sodiumchloride, potassiumchloride, sodiumsulphate, sodiumsulphite, magnesiumchloride, magnesium(bi)-carbonate, ammoniumchloride, sodiumglycolate, but also chloride-, sulphate-, phosphate- and carbamate salts of amines such as, for instance, isopropylamine and alkanolamines. In addition, it has been found that excellent results can be achieved also with urea and thiourea.

Preference is given to using sodium-, magnesium- or ammonium salts or urea. Mixtures of such compounds can be used also, without real limitations as to their relative proportions.

In order to obtain a definite dioxane-reducing effect, the amount of the compound(s) present in the sulphation process must at least be 0.5% (wt) calculated on the starting compound (ethoxylated alcohol) to be sulphated. Good results are obtained by using amounts of 0.5-15% (wt) of the said compound(s) calculated on the etheralcohol. In determining the optimum amount the person skilled in the art will, of course, always choose such an amount as to keep the reaction mixture capable of being stirred. The practical upper limit (in that respect) is at different levels, depending on the dioxane-suppressing compound used. Of course, it also depends on the nature of the ethoxylated alcohol itself that is to be sulphated. The amount of salt that can be used is usually (on a weight basis) lower than that of urea. Ammonium salts, too, for instance, can be used in relatively high concentrations. All this, however, can easily be calculated by the person skilled in the art.

The best results are achieved when, before its addition to the etheralcohol or to the reaction mixture, the dioxane-suppressing compound is brought into a finely distributed state by grinding.

The process according to the invention can be applied in the sulphation of a variety of ethoxylated alcohols by means of chlorosulphonic acid. The etheralcohols to be sulphated may be derived from saturated as well as unsaturated aliphatic alcohols, which may be branched or unbranched, but also from fatty acid alkanolamides. If etheralcohols derived from natural fatty alcohols are started from, the alcohol residue is usually a mixture of various groups having different chain lengths. The etheralcohols may be derived also from polyols such as, for instance, glucose. The average number of oxyethylene groups in the etheralcohol may range from 0.5 to 20, or even higher. The ethersulphates as used in detergents and cosmetic products are usually derived from fatty alcohols with 6-20 C-atoms and contain on average 0.5-8 oxyethylene groups. The (exothermic) sulphation is preferably carried out at temperatures lower than 50¤C. It has been found that, when cooling with cooling water, temperatures of between 30 and 40oC can be set and that in the process according to the invention excellent results can be achieved with these. When temperatures are lower still, which can be achieved by using a different cooling system, for instance a deep-cooling unit, even lower dioxane percentages are reached. In this embodiment, however, the production costs show a substantial increase. Usally, extra investments will be necessary, too, to make the equipment suited for the lower temperatures. The lower limit of the reaction temperature is in practice determined also by such factors as the solidification of the ethoxylated alcohol and/or the crystallization of the reaction product.

Although in this application exclusive rights are applied for only in respect of the preparation of ethersulphates by sulphation of ethoxylated alcohols using chlorosulphonic acid, it is assumed that the use of dioxane-suppressing compounds according to the invention can successfully be applied also in the $SO_3$ process.

The invention is further elucidated by the following experiments without, however, being limited thereto. The experiments were carried out according to either of the embodiments described below, which, on the one side, differ in the nonionic used and, on the other, result in dilute, respectively concentrated, products in the neutralization stage. The results are summarized in the form of a table, stating each time what dioxane-suppressing compound was used and in what amount. For the purpose of comparison, the tables also include the experiments carried out without dioxane-suppressing compounds under otherwise identical conditions.

Embodiment 1

Preparation of dilute sodiumethersulphate solutions

In a 2-litre four-neck flask provided with a thermometer, a stirrer, a 250 ml drop funnel and a tubular outlet for the escape of gaseous HCl was presented each time 429 grammes dioxane-free Lorol 1216[R] ethoxylated with, on average, 2 ethylene oxide groups per mole. Lorol 1216[R] is a mixture of substantially laurylalcohol (c. 60%), myristylalcohol (c. 25%) and cetylalcohol (c. 10%); available from Henkel. The OH number of these 429 grammes of starting product was 199 mg KOH/g, i.e. 1525 meq OH groups in all.

To the 429 grammes of starting product one (or more) dioxane-suppressing compound(s) was (were) added each time in a finely distributed state, as indicated in table 1. The mixture was subsequently stirred for 15 minutes and cooled to 12¤C in an ice/water bath. Subsequently, during stirring and continued cooling, 180.65 grammes (1551 mmoles) chlorosulphonic acid was added in drops in 50 minutes, during which addition the temperature of the reaction mixture in the first instance rose to 40¤C and was subsequently maintained at this level by adjusting the metering rate till about 70% of the chlorosulphonic acid had been metered. After that, the temperature was lowered to 32¤C by a short interruption of the metering. After the rest of the chlorosulphonic acid had been added at this temperature, $N_2$ gas was passed through the reaction mixture at the same temperature for 15 minutes more for the purpose of removing any residual hydrochloric acid gas.

Subsequently, in an $N_2$ atmosphere, the resulting reaction product was poured out for neutralization purposes in 15 minutes into a 3-litre beaker containing a solution of 62.5 grammes NaOH in 1465 grammes water. During this process the neutralized product was kept alkaline (at pH = 8.0) by metering a (measured) amount of a 50% NaOH solution in water. A total amount of c. 150 grammes of it proved to be necessary each time. The resulting solution of the sodium salt of the ethersulphate was subsequently stirred to perfectly homogeneous and preserved by adding 1 gramme of a commercially available solution Euxyl L 400.

Of the resulting product the Epton value was determined in meq/gramme ethersulphate (calculated as sodium salt) by titration with cetylpyridiniumchloride, from which value the conversion percentage can be calculated. The dioxane content (in ppm) was determined gaschromatographically and subsequently converted to the content calculated on 100% DS. The results are mentioned in table 1.

Embodiment 2

Preparation of concentrated sodiumethersulphate solutions

In the same arrangement as described in embodiment 1, 425.2 grammes dioxane-free Dobanol 23[R] ethoxylated with on average 2 ethylene oxide groups per mole was presented each time. Dobanol 23[R] is a mixture of partly branched $C_{12}$ and $C_{13}$ alcohols (available from Shell).

The OH number of these 425.2 grammes of starting product was 204 mg KOH/g, i.e. 1546 meq OH groups in all. To the 425.2 grammes of starting product a dioxane-suppressing compound was added each time, as indicated in table 2, upon which, in the same way as in embodiment 1, each time 185.5 grammes (1592 mmoles) chlorosulphonic acid was metered and $N_2$ gas was passed through. In an $N_2$ atmosphere the resulting reaction product was subsequently poured out into a 3-litre beaker containing a solution of 71.2 grammes NaOH and 14.9 grammes NaCl in 425 grammes water, the pH of the neutralized product being

4

kept at 8.0 by addition of a (measured) amount of a 50% NaOH solution in water.

By titration with cetylpyridiniumchloride, the Epton value of the resulting product was determined in meq/gramme of ether-sulphate (calculated as sodium salt) for the purpose of calculating the degree of conversion in %. The dioxane content was determined gaschromatographically and subsequently converted to the content calculated on 100% DS. The results are mentioned in table 2.

Table 1

| Nonionic: ethoxylated Lorol 1216; further processing into dilute salt solution. | | | | |
|---|---|---|---|---|
| | A | B | C | D |
| Exp | Dioxane-suppressing compound | Amount A (in %(wt) on nonionic) | Conversion of nonionic into ethersulphate (in %) | Dioxane in end product on DS (ppm/100% DS) |
| 1.1 | ammoniumchloride | 1 | 99.5 | 35 |
| 1.2 | ammoniumchloride | 5 | 99.3 | 10 |
| 1.3 | sodiumchloride | 1.25 | 98.1 | 25 |
| 1.4 | sodiumglycolate | 1.5 | 96.1 | 61 |
| 1.5 | ammoniumcarbonate | 1.28 | 95.2 | 69 |
| 1.6 | ammoniumcarbonate | 15 | 95 | 14 |
| 1.7 | magnesiumoxide | 0.8 | 97.6 | 68 |
| 1.8 | diethylamine | 15 | 97 | 29 |
| 1.9 | monoethanolamine | 1 | 92.3 | 72 |
| 1.10 | thiourea | 5 | 96.6 | 63 |
| 1.11 | urea | 1 | 98.5 | 79 |
| 1.12 | urea | 5 | 97.3 | 27 |
| 1.13 | urea | 10 | 95.5 | 8 |
| 1.14 | urea + sodiumchloride | 5 1 | 92.2 | 16 |
| 1.15 | urea + ammoniumchloride | 5 1 | 94.4 | 27 |
| Comparative experiment No dioxane-suppressing compound | | | 99.1 | 138 |

Table 2

| Nonionic: ethoxylated Dobanol 23; further processing to concentrated salt solution. | | | | |
|---|---|---|---|---|
| | A | B | C | D |
| Exp no. | Dioxane-suppressing compound | Amount (in %(wt) on nonionic) | Conversion of nonionic to ethersulphate (in %) | Dioxane in end product on DS (ppm/100% DS) |
| 2.1 | sodiumchloride | 1.66 | 100 | 32 |
| 2.2 | sodiumchloride (coarse) | 1.66 | 99.8 | 59 |
| 2.3 | sodiumcarbonate | 1.5 | 96.2 | 28 |
| 2.4 | sodiumphosphate | 1.55 | 99.2 | 42 |
| 2.5 | lithiumcarbonate | 1.05 | 97.9 | 42 |
| Comparative experiment No dioxane-suppressing compound | | | 99.5 | 110 |

**Claims**

1. Process for preparing ethersulphates by virtually complete sulphation of ethoxylated alcohols, using chlorosulphonic acid, and subsequent neutralization, characterized in that the sulphation is carried out at least from a degree of conversion of 60% in the presence of one or more dioxane-suppressing compound-(s) from the group of (thio)urea and chlorides, sulphates, sulphites, phosphates, (bi)carbonates, glycolates or carbamates of (earth) alkali metals, ammonium or alkyl- and/or alkanolamines in an amount of at least 0.5% (wt) calculated on the amount of ethoxylated alcohol initially present.

2. Process according to claim 1, characterized in that the dioxane-suppressing compound(s) is (are) added to the ethoxylated alcohol before commencement of the sulphation.

3. Process according to any one of claims 1-2, characterized in that the dioxane-suppressing compound used is one or more sodium-, magnesium- or ammonium salt(s) and/or urea.

4. Process according to any one of claims 1-3, characterized in that the dioxane-suppressing compound is used in an amount of 0.5-15% (wt) calculated on the amount of ethoxylated alcohol initially present.

5. Process according to any one of claims 1-4, characterized in that the dioxane-suppressing compound is applied in a finely distributed state.

European Patent
Office

EUROPEAN SEARCH REPORT

Application Number

EP 89 20 3154

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-2 928 860 (R.C. HARRINGTON) <br> * Claims * <br> --- | 1-4 | C 07 C 305/10 <br> C 07 C 303/24 |
| X | US-A-3 332 979 (C.E. REDEMANN) <br> * Claims * <br> --- | 1,3 | |
| X | EP-A-0 052 801 (HENKEL) <br> * Page 2, paragraph 3 * <br> ----- | 1 | |

TECHNICAL FIELDS
SEARCHED (Int. Cl.5)

C 07 C 303/00
C 07 C 305/00

The present search report has been drawn up for all claims

| Place of search | Date of completion of the search | Examiner |
|---|---|---|
| THE HAGUE | 16-03-1990 | VAN GEYT J.J.A. |

EPO FORM 1503 03.82 (P0401)